# EUROPÄISCHE PATENTSCHRIFT

(11) **EP 0 612 745 B1**
(45) Veröffentlichungstag und Bekanntmachung des Hinweises auf die Patenterteilung: **09.12.1998**
(21) Anmeldenummer: 94101043.1
(22) Anmeldetag: 25.01.1994
(51) Int. Cl.: C07D 487/04

(54) **Verfahren zur Herstellung von 1,2,3,4,10,14b-Hexahydro-2-methyl-dibenzo[c,f]pyrazino[1,2-a]azepin und seiner Salze**
Process for the preparation of 1,2,3,4,10,14b-hexahydro-2-methyldibenzo[c,f ]pyrazino[ 1,2-a] azepin and their salts
Procédé pour la préparation de 1,2,3,4,10,14b-hexahydro-2-méthyldibenzo[ c,f ]pyrazino[1,2-a]- azépine et leur sels

(30) Priorität: 24.02.1993 DE 4305659
(43) Veröffentlichungstag der Anmeldung: 31.08.1994
(73) Patentinhaber: HEUMANN PHARMA GmbH, D-90478 Nürnberg (DE)
(72) Erfinder: Kisielowski-Ruppert, Lothar Dr. Dipl. Chem., D-90409 Nürnberg (DE); Mörsdorf, Johann Peter Dr. Dipl. Chem., D-90579 Langenzenn (DE); Grafe, Ingomar Dr. Dipl. Chem., D-90482 Nürnberg (DE); Ahrens, Kurt-Henning Dr., D-90403 Nürnberg (DE)
(74) Vertreter: Kraus, Walter, Dr.

(56) Entgegenhaltungen:
- DE-A- 2 505 239

## Beschreibung

Die Erfindung betrifft ein neues Verfahren zur Herstellung von 1,2,3,4,10,14b-Hexahydro-2-methyl-dibenzo[c,f]pyrazino[1,2-a]azepin. Diese Verbindung ist bereits aus der DE-A 16 95 556 bekannt und hat unter dem INN-Namen Mianserin Eingang in die Therapie depressiver Erkrankungen gefunden.

Bei dem in der DE-A 16 95 556 beschriebenen Verfahren erfolgt die Synthese von Mianserin ausgehend von Morphanthridinderivaten der Formel A 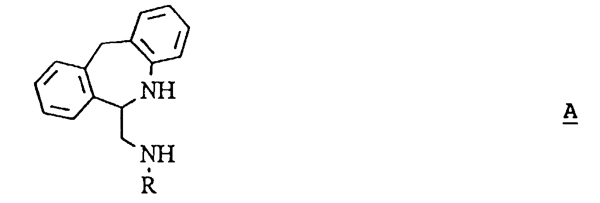 (worin R eine niedere Alkylgruppe bedeutet), die ihrerseits nur schwer über vielstufige Synthesen zugänglich sind. Die Morphanthridinderivate der Formel A werden dabei in einer mehrstufigen, sehr aufwendigen Reaktionsfolge zu Diketopiperazinen umgesetzt, die zu Mianserin reduziert werden. Diese Reduktion gelingt nur mit problematisch zu handhabenden Reagentien, wie Diboran oder Lithiumaluminiumhydrid. Die Ausbeute des gesamten Verfahrens ist äußerst gering und liegt unter 10%.

Die DE-A 25 05 239 beschreibt ein verbessertes Verfahren zur Herstellung von Mianserin und analogen Verbindungen. Bei diesem bekannten Verfahren erfolgt die Umsetzung gemäß folgendem Reaktionsschema:

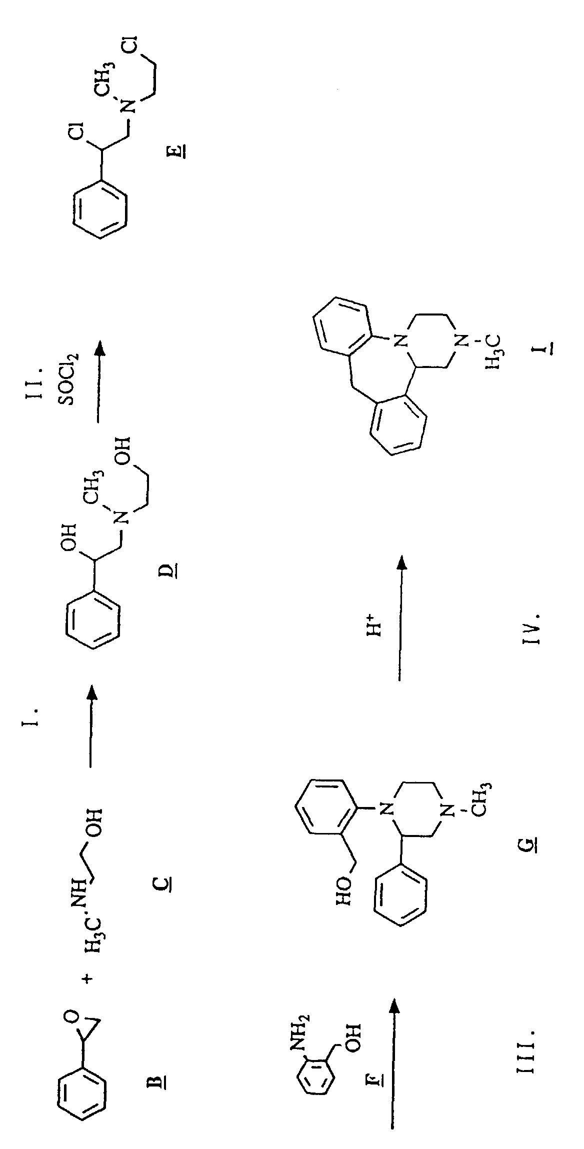

In der ersten Stufe des bekannten Verfahrens wird Styroloxid B mit Methylaminoethanol C zum Diol D umgesetzt. Dieses wird dann in der zweiten Stufe mit Thionylchlorid zum Dichlorid E chloriert. Das so erhaltene Dichlorid E wird hierauf in der dritten Stufe mit 2-Aminobenzylalkohol F zum Piperazin G cyclisiert. Das Piperazin G wird dann in der vierten Stufe durch Umsetzung mit einer starken Säure, wie zum Beispiel konzentrierte Schwefelsäure, unter einem weiteren Ringschluß zu Mianserin der Formel (I) umgesetzt. Nach den Angaben der DE-A 25 05 239 soll die Gesamtausbeute dieses Verfahrens bei 38% liegen.

Der Erfindung liegt die Aufgabe zugrunde, die Nachteile der bekannten Verfahren zu überwinden und insbesondere ein neues Verfahren zur Herstellung von Mianserin bereitzustellen, das diese Verbindung mit der gleichen Qualität wie die bekannten Verfahren aber mit deutlich verbesserter Ausbeute liefert.

Diese Aufgabe wird erfindungsgemäß dadurch gelöst, daß
(a) Styroloxid der Formel (II) 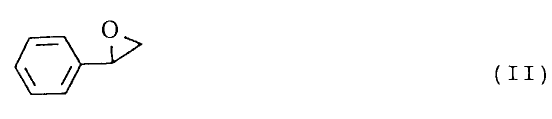 mit N-Benzylaminoethanol der Formel (III) 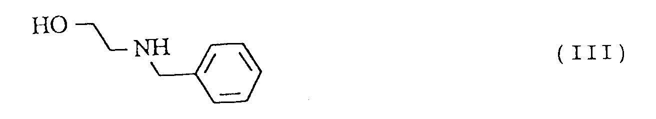 bei einer Reaktionstemperatur von 80 bis 160°C zu der Verbindung der Formel (IV) 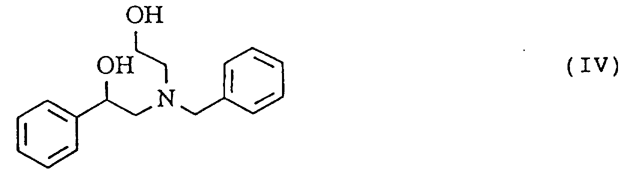 umgesetzt wird,
(b) die erhaltene Verbindung der Formel (IV) in einem inerten aprotischen Lösungsmittel mit einem Chlorierungsmittel in die Verbindung der Formel (V) 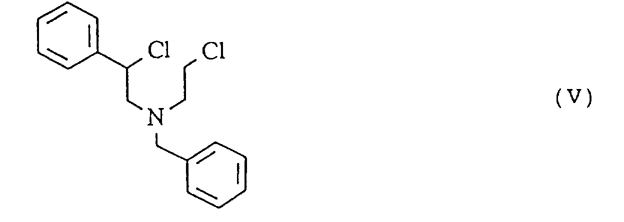 überführt wird und die resultierende Verbindung der Formel (V) ohne Isolierung der Verbindung der Formel (V) mit 2-Aminobenzylalkohol der Formel (VI) 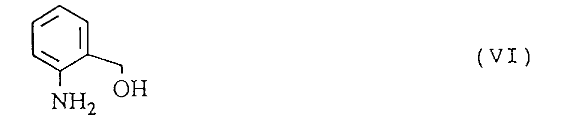 zu der Verbindung der Formel (VII) 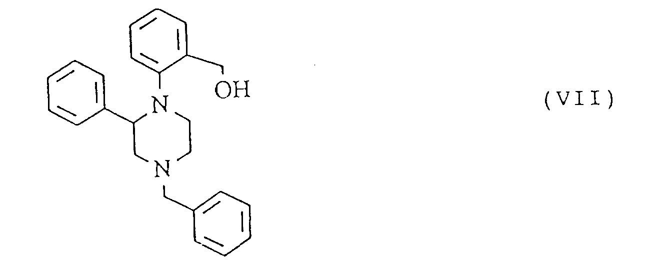 kondensiert wird, wobei das Molverhältnis des 2-Aminobenzylalkohols der Formel (VI) zu der Verbindung der Formel (V) 0,8:1 bis 1,2:1 beträgt, wobei die unter Cyclisierung erfolgende Kondensation in einem Zweiphasensystem erfolgt, wobei dem Reaktionsgemisch ein entsprechendes Äquivalent Base zur Bindung der bei der Reaktion entstehenden Säure zugesetzt wird und wobei die Isolierung der Verbindung der Formel (VII) durch Kristallisation eines geeigneten Salzes erfolgt,
(c) die erhaltene Verbindung der Formel (VII) mit Hilfe einer starken Säure, die in großem molaren Überschuß eingesetzt wird, zu der Verbindung der Formel (VIII) 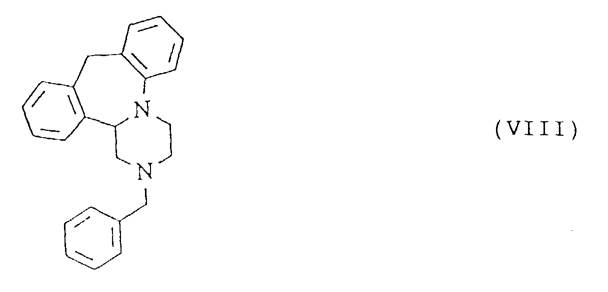 cyclisiert wird, wonach das zunächst gebildete Salz der verbindung der Formel (VIII) zur Bildung der freien Base der Verbindung (VIII) mit einer geeigneten Base umgesetzt wird,
(d) in der erhaltenen Verbindung der Formel (VIII) die Benzylgruppe durch Umsetzung mit einem molaren Überschuß eines Chlorameisensäureesters der Formel (IX) 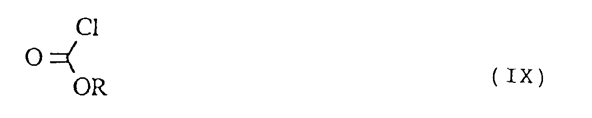 worin R für eine C₁-C₆-Alkylgruppe, eine Phenyl- oder Benzylgruppe steht, in einem aprotischen organischen Lösungsmittel entfernt und die intermediär gebildete Verbindung der Formel (X) 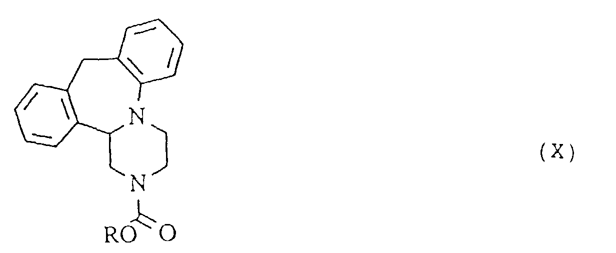 mit einer Base zu der Verbindung der Formel (XI) 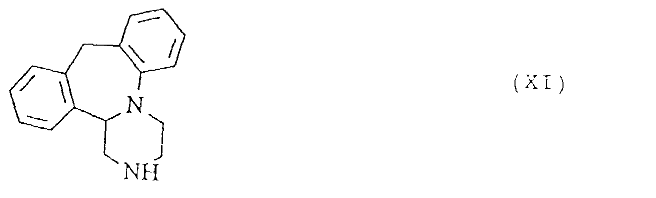 verseift wird,
(e) die resultierende Verbindung der Formel (XI) unter reduzierenden Bedingungen mit Formaldehyd durch katalytische reduktive Aminierung mit Wasserstoff zu 1,2,3,4,10,14b-Hexahydro-2-methyl-dibenzo[c,f]pyrazino[1,2-a]azepin der Formel (I) methyliert wird, und
(f) gegebenenfalls die in Stufe (e) erhaltene Base in an sich bekannter Weise in ihr physiologisch annehmbares Salz umgewandelt wird.

Bei dem erfindungsgemäßen Verfahren wird durch Verwendung eines speziellen Ausgangsmaterials und eines daran angepaßten Synthesewegs der Nachteil des Verfahrens der DE-A 25 05 239, daß schon in der ersten Synthesestufe ein unerwünschtes Isomeres gebildet wird, überwunden. Bei dem bekannten Verfahren wird nämlich in der ersten Stufe, d.h. bei der Umsetzung von Styroloxid mit N-Methylaminoethanol neben der gewünschten Verbindung D durch Ringöffnung des Epoxidrings von B am zweiten Kohlenstoffatom ein zweites Isomeres der Formel H gebildet. Dieses Isomere entsteht bei dem bekannten Verfahren mit einer Ausbeute von etwa 25% und es reagiert nicht in der gewünschten Weise zu Mianserin weiter. Hierdurch wird eine erhebliche Verschlechterung der Gesamtausbeute des bekannten Verfahrens bewirkt.

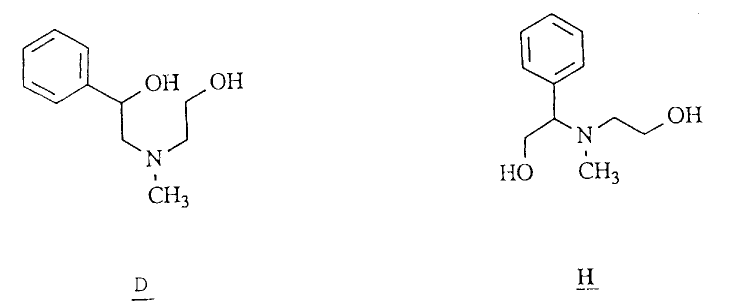

Wie oben bereits zum Ausdruck gebracht wird bei dem Verfahren gemäß der Erfindung im wesentlichen kein unerwünschtes Isomeres gebildet, so daß die weitere Umsetzung praktisch ausschließlich mit der Verbindung (IV) erfolgt.

Im folgenden wird das erfindungsgemäße Verfahren anhand der einzelnen Stufen näher beschrieben:

### 1. Stufe (a)

In der ersten Stufe wird Styroloxid der Formel (II) mit N-Benzylaminoethanol der Formel (III) zum Zwischenprodukt der Formel (IV) umgesetzt. 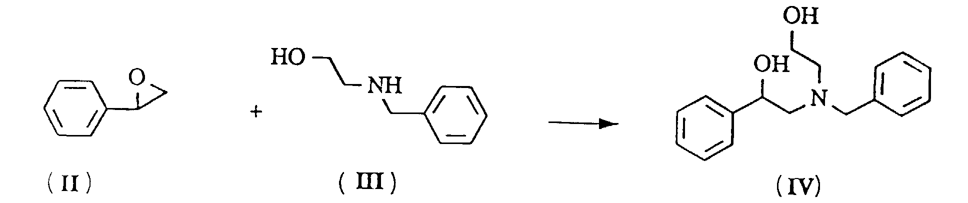 Die Umsetzung wird vorzugsweise bei Atmosphärendruck in äquimolaren Mengenverhältnissen und in Anwesenheit oder Abwesenheit eines Lösungsmittels durchgeführt, wobei die Umsetzung in Abwesenheit von Lösungsmittel bevorzugt wird. Geeignete Lösungsmittel sind zum Beispiel aromatische Kohlenwasserstoffe wie Xylol oder Mesitylen.

Die Reaktionstemperatur beträgt beispielsweise 80 bis 160°C, vorzugsweise 100 bis 140°C. Das Produkt der Formel (IV) wird als viskoses Öl erhalten und kann ohne Reinigung in die nächste Stufe eingesetzt werden. Nach dem ¹H-NMR-Spektrum enthält das Produkt maximal 3% des unerwünschten, zur Verbindung H analogen Isomeren.

### 2. Stufe (b)

Die Verbindung der Formel (IV) wird mit einem Chlorierungsmittel in das Dichlorid der Formel (V) übergeführt. 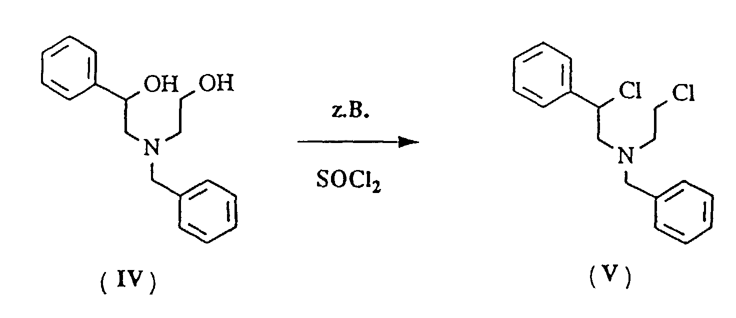 Zur Umsetzung der Verbindung (IV) zur Dichlorverbindung der Formel (V) sind die üblichen Chlorierungsmittel, wie Thionylchlorid, Sulfurylchlorid oder Phosphoroxidchlorid geeignet, wobei Thionylchlorid bevorzugt wird. Das Molverhältnis der Verbindung der Formel (IV) zum Chlorierungsmittel kann 1:2 bis 1:4 betragen, wobei ein Molverhältnis von 1:2,1 bis 1:2,3 bevorzugt wird. Die Reaktion wird in einem inerten, aprotischen Lösungsmittel, wie zum Beispiel chlorierten Kohlenwasserstoffen, wie Dichlorethan oder Chlorbenzol, oder Estern wie zum Beispiel Ethylacetat, und bei Temperaturen von Raumtemperatur bis zur Siedetemperatur des verwendeten Lösungsmittels durchgeführt. Bevorzugte Reaktionsbedingungen sind Chlorbenzol als Lösungsmittel und ein Temperaturbereich von 60 bis 80°C.

Die Verbindung der Formel (V) wird ohne Isolierung mit 2-Aminobenzylalkohol (VI) in Gegenwart einer Base zum Piperazin der Formel (VII) cyclisiert. 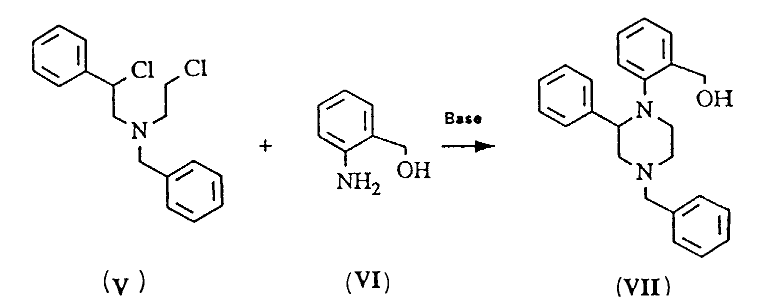 Der Aminobenzylalkohol der Formel (VI) wird dabei im Molverhältnis 0,8:1 bis 1,2:1, bezogen auf die Verbindung der Formel (V), eingesetzt, wobei ein Unterschuß von Aminobenzylalkohol, d.h. ein Molverhältnis von 0,8:1 bis 1:1 bevorzugt wird. Die Reaktion kann im Temperaturbereich von 20°C bis zum Siedepunkt des Reaktionsgemisches geführt werden, wobei ein Temperaturbereich von 50 bis 100°C vorteilhaft ist.

Die unter Cyclisierung erfolgende Kondensation der Verbindung (V) mit 2-Aminobenzylalkohol (VI) kann in polaren organischen Lösungsmitteln oder in organisch-wäßrigen Zweiphasensystemen unter Phasentransferbedingungen durchgeführt werden. Geeignete polare organische Lösungsmittel sind beispielsweise Alkohole wie Butanol oder Etheralkohole wie die Polyethylenglykole, zum Beispiel Polyethylenglykol 400. Bevorzugt ist jedoch eine zweiphasige Arbeitsweise unter Phasentransferbedingungen. Dabei finden insbesondere Chlorkohlenwasserstoffe, bevorzugt Chlorbenzol, als organische Phase Verwendung.

Zur Bindung der bei der Reaktion entstehenden Säure wird dem Reaktionsgemisch ein entsprechendes Äquivalent Base zugesetzt. Geeignet sind bei Verwendung der einphasigen Arbeitsweise Alkalialkoholate wie Natriummethylat oder Alkalihydroxide wie Natriumhydroxid und im Fall der zweiphasigen Arbeitsweise Alkali- oder Erdalkalihydroxide, wie Natrium-, Kalium- oder Calciumhydroxid, oder Alkalicarbonate wie Kaliumcarbonat.

Als Phasentransferkatalysatoren können die üblichen quartären Ammoniumsalze, wie beispielsweise Tetrabutylammoniumsulfat oder Benzyltriethylammoniumchlorid verwendet werden.

Die Isolierung der Verbindung der Formel (VII) erfolgt vorzugsweise durch Kristallisation eines geeigneten Salzes. Dabei ist das stabile und gut kristallisierende Fumarat besonders bevorzugt.

### 3. Stufe (c)

In einer zweiten Cyclisierung wird das Piperazinderivat der Formel (VII) mit einer starken Säure zu der tetracyclischen Verbindung (VIII) umgesetzt. 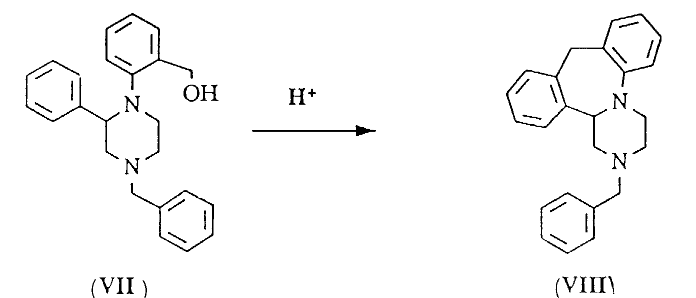 Als Säuren eignen sich dabei insbesondere solche Säuren, die zusätzliche wasserentziehende Eigenschaften haben, wie Schwefelsäure, Phosphorsäure oder Polyphosphorsäure, wobei 70%- bis 100%ige Schwefelsäure bevorzugt wird. Die Säure wird dabei grundsätzlich in großem molaren Überschuß eingesetzt, wobei die untere Grenze durch die Rührfähigkeit des Gemisches vorgegeben ist. Die Umsetzung wird vorteilhafterweise im Temperaturbereich von 20 bis 80°C geführt. Das zunächst gebildete Salz der Verbindung der Formel (VIII) wird für die Umsetzung in der nächsten Stufe mit einer geeigneten Base umgesetzt, um die freie Base der Verbindung (VIII) zu erhalten. Geeignete Basen sind zum Beispiel Alkalihydroxid wie Natronlauge.

### 4. Stufe (d)

In dieser Stufe wird die Verbindung der Formel (VIII) debenzyliert, indem durch Umsetzung mit einem Chlorameisensäureester der Formel (IX), in der R für eine C₁-C₆-Alkylgruppe, eine Phenyl- oder eine Benzylgruppe steht, die Benzylgruppe durch eine Urethangruppierung ersetzt wird. Das intermediär gebildete Urethan der Formel (X) wird dann basisch zur Verbindung der Formel (XI) verseift. 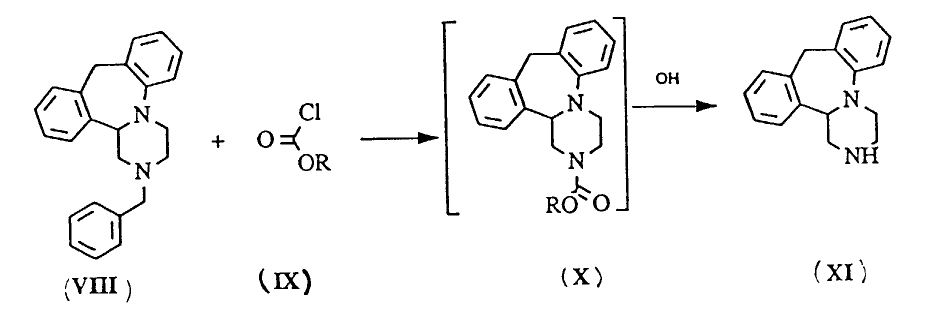 Die Umsetzung der Verbindung (VIII) mit dem Chlorameisensäureester der Formel (IX) erfolgt in einem aprotischen organischen Lösungsmittel und mit einem molaren Überschuß des Chlorameisensäureesters. Bevorzugte Lösungsmittel für diese Umsetzung sind Kohlenwasserstoffe, wie Toluol oder Xylol, oder höhersiedende Ketone, wie beispielsweise Methylisobutylketon. Von den Chlorameisensäureestern wird der Ethylester und insbesondere der Butylester bevorzugt. Die Umsetzung wird vorzugsweise bei einem Molverhältnis von 1:1,1 bis 1:1,2 und bei erhöhter Temperatur, insbesondere im Temperaturbereich von 70 bis 130°C durchgeführt.

Für die Verseifung des intermediär gebildeten Urethans der Formel (X) werden Alkohole als Lösungsmittel bevorzugt. Bei einer bevorzugten Ausführungsform dieses Schrittes wird die Verbindung (X) mit Kaliumhydroxid als Base in n-Butanol bei Rückflußtemperatur umgesetzt. Andere Alkali- und Erdalkalihydroxide sind als Basen jedoch gleichermaßen geeignet. Die Reaktion wird vorzugsweise bei höheren Temperaturen, d.h. bei Rückflußtemperatur des verwendeten Alkohols, im bevorzugten Fall der Verwendung von Butanol bei 120°C, durchgeführt. Bei Verwendung von Alkoholen mit Siedepunkten unter 100°C kann die Reaktion zur Erreichung einer höheren Reaktionstemperatur auch unter Druck in geschlossenen Gefäßen durchgeführt werden.

### 5. Stufe (e)

Die nach der Verseifung der Verbindung der Formel (X) erhaltene Verbindung der Formel (XI) wird gegebenenfalls ohne Isolierung im abschließenden Schritt reduktiv mit Formaldehyd zu der Titelverbindung der Formel (I) methyliert. Diese mit Formaldehyd verlaufende Methylierung kann entweder nach Leuckart-Wallach mit Ameisensäure als Reduktionsmittel oder als katalytische reduktive Aminierung mit Wasserstoff erfolgen.

Insbesondere die katalytische reduktive Aminierung verläuft überraschend einheitlich und sauber, die Entstehung von Nebenprodukten wird nicht beobachtet. 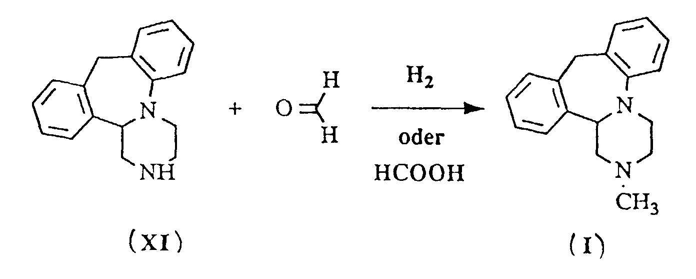 Bei der bevorzugten Ausführungsweise wird die Verbindung der Formel (XI) in einem geeigneten Lösungsmittel mit Formaldehyd und Wasserstoff in Gegenwart eines Hydrierungskatalysators und gegebenenfalls unter zusätzlicher Katalyse mit einer Säure umgesetzt. Als Lösungsmittel eignen sich polare, organische Lösungsmittel, insbesondere Alkohole, wie Ethanol, Isopropanol oder n-Butanol, wobei n-Butanol bevorzugt wird. Die Umsetzung mit Formaldehyd, der bevorzugt in Form einer handelsüblichen wäßrigen Lösung ("Formalin-Lösung") eingesetzt wird, wird bei Temperaturen von 10 bis 80°C und mit einem molaren Überschuß an Formaldehyd durchgeführt. Dabei wird die 1,5- bis 4fache molare Menge an Formaldehyd eingesetzt, wobei die ungefähr 2fache molare Menge bevorzugt ist.

Die Reduktion des intermediär gebildeten Halbaminals mit Wasserstoff wird ohne Isolierung der Verbindung und vorzugsweise im gleichen Lösungsmittel bei Temperaturen von 20 bis 100°C und bei Wasserstoffdrücken von 1 bis 10 bar durchgeführt. Als Katalysatoren können die üblichen Hydrierungskatalysatoren wie Platin, Palladium, Rhodium oder Raney-Nickel, gegebenenfalls auf einem geeigneten Trägermaterial wie Aktivkohle, Verwendung finden.

Vorteilhafterweise werden zum Reaktionsgemisch zur Reaktionsbeschleunigung auch katalytische Mengen einer Säure, beispielsweise Salzsäure oder Schwefelsäure, gegeben.

Die reduktive Aminierung nach Leuckart-Wallach wird unter an sich bekannten Bedingungen mit einem Überschuß von Ameisensäure und bei erhöhter Temperatur durchgeführt. Vorzugsweise dient dabei der Überschuß an Ameisensäure als Lösungsmittel. Die Reaktion wird im Temperaturbereich von 40 bis 80°C, zur Unterdrückung von Nebenprodukten bevorzugt bei 40 bis 60°C, durchgeführt.

Abschließend kann die erhaltene Mianserin-Base nach an sich bekannten Methoden in ein physiologisch annehmbares Salz, vorzugsweise das Hydrochlorid, übergeführt werden. So wird nach dem erfindungsgemäßen Verfahren Mianserin-Hydrochlorid der Formel (I) mit gleicher Reinheit wie bei dem Verfahren der DE-A 25 05 239, aber mit einer deutlich verbesserten Gesamtausbeute von 54% erhalten.

Das nachfolgende Beispiel erläutert die Erfindung:

### Beispiel

### 1. Herstellung von N-Benzyl-N-(2-hydroxy-2-phenyl-ethyl)-2-aminoethanol [Verbindung (IV)]

In einem 2-1-Dreihalskolben werden 907 g (862 ml) N-Benzylaminoethanol vorgelegt und auf 100°C erwärmt. Von den insgesamt benötigten 721 g (685 ml) Styroloxid werden zunächst 50 ml in einer Portion zulaufen gelassen. Die Temperatur fällt dabei auf ungefähr 95°C, steigt dann aber wieder allmählich an. Sobald die Temperatur ansteigt, wird das restliche Styroloxid zugetropft. Die Umsetzung verläuft exotherm. Die Zutropfgeschwindigkeit wird so eingestellt, daß die Innentemperatur im Bereich von 120 bis 140°C liegt.

Nach beendigter Styroloxid-Zugabe wird das Reaktionsgemisch eine Stunde lang bei 130°C gehalten. Flüchtige Komponenten können anschließend durch vorsichtiges Anlegen von Vakuum aus dem heißen Reaktionsgemisch entfernt werden. Es wird ein hochviskoses, farbloses bis gelbliches Öl erhalten.

Eine weitere Reinigung des Produkts ist nicht nötig. Die Ausbeute ist quantitativ. Das ¹H-NMR-Spektrum zeigt die Anwesenheit von 2 bis 3% des unerwünschten Isomeren.

### 2. Herstellung von 4-Benzyl-l-(2-hydroxymethylphenyl)-2-phenylpiperazin-fumarat (1:1) [Fumarat der Verbindung (VII)]

### 2.1 Herstellung des Dichlorids der Formel (V)

421 g (1,55 mol) des in 1. erhaltenen Benzyldiolamins werden unter Rühren in 1000 ml Chlorbenzol gelöst und mit 3 g 4-Dimethylaminopyridin versetzt. Die Lösung wird auf 60°C erhitzt.

Anschließend werden 248 ml Thionylchlorid zugetropft. Nach vollständiger Zugabe des Thionylchlorids wird noch 40 Minuten lang bei 70°C gerührt. Die zunächst kräftige Gasentwicklung läßt allmählich nach. Man entfernt das Heizbad, spült zunächst 20 Minuten lang mit Stickstoff und kühlt dann im Eisbad auf 5°C ab. Zur Vernichtung von überschüssigem Thionylchlorid werden 300 ml Wasser so zugetropft, daß die Temperatur 25°C nicht überschreitet. Anschließend werden ungefähr 390 ml 30%iger Natronlauge ebenfalls so zugetropft, daß die Temperatur 25°C nicht überschreitet. Es muß ein pH-Wert von 5 bis 6 eingestellt werden.

### 2.2 Umsetzung mit 2-Aminobenzylalkohol

In einem 4-l-Vierhalskolben werden 428 g Kaliumcarbonat in 400 ml Wasser unter Rühren im Eisbad gelöst. Die erhaltene Lösung wird mit 10 g Tetrabutylammoniumsulfat, 100 ml Chlorbenzol und 153 g Aminobenzylalkohol versetzt. Bei einer Anfangstemperatur von ungefähr 20°C wird zu dieser Suspension unter kräftigem Rühren zügig das in 2.1 erhaltene zweiphasige Gemisch des Dichlorids zulaufen gelassen. Das erhaltene Gemisch wird zunächst langsam auf 40 bis 50°C erhitzt, wobei der Aminobenzylalkohol in Lösung geht. Danach wird bis zum Rückfluß (Innentemperatur ungefähr 97°C) erhitzt und das Reaktionsgemisch 3 Stunden lang unter Rückfluß gehalten. Nach dem Abkühlen auf ungefähr 90°C werden die Phasen getrennt. Die untere wäßrige Phase wird verworfen.

Die Chlorbenzol-Lösung wird im Vakuum bis zu einer Sumpftemperatur von 80°C eingeengt. Der braune ölige Rückstand wird in 700 ml Aceton aufgenommen, ungelöste Salze werden abfiltriert (Supercel) und der Kolben und Nutschen werden mit 200 ml Aceton nachgespült.

### 2.3 Fällung des Fumarats der Verbindung (VII)

In einem 4-l-Vierhalskolben werden 144 g Fumarsäure in 400 ml Aceton suspendiert und unter Rühren zum Rückfluß erhitzt. Zu dieser Suspension läßt man die unter 2.2 erhaltene Aceton-Lösung der Piperazin-Base bei Rückfluß fließen, worauf das Fumarat auskristallisiert.

Die Suspension wird weitere 15 Minuten lang unter Rückfluß erhitzt, dann zunächst mit kaltem Wasser, anschließend im Eisbad auf 5 bis 10°C abgekühlt.

Der Niederschlag wird über ein Filtertuch abgesaugt und mit Aceton gewaschen bis das Filtrat und das Produkt fast farblos sind (2 Sickerwäschen mit 400 ml Aceton). Das Fumarat wird gut trockengesaugt und im Vakuum 4 Stunden lang bei 80°C getrocknet. Es werden farblose Kristalle vom Schmelzpunkt 200 bis 202°C erhalten.

Ausbeute: 468 g (79% d. Th.) bezogen auf Aminobenzylalkohol.

### 3. Herstellung von 1,2,3,4,10,14b-Hexahydro-2-benzyl-dibenzo[c,f]pyrazino[1,2-a]azepin [Verbindung (VIII)]

### 3.1 Cyclisierung

In einem 2-l-Dreihalskolben wird 1 l Schwefelsäure (96%) vorgelegt und im Eisbad auf ungefähr 13 bis 15°C gekühlt. Unter Eiskühlung und Rühren werden portionsweise 712 g (1,5 mol) 2-Hydroxymethylphenylpiperazin-fumarat eingetragen. Nach beendigter Fumarat-Zugabe wird die Suspension zunächst 30 Minuten ohne Kühlung gerührt. Dann wird die Suspension auf ungefähr 70 bis 80°C erhitzt, eine Stunde lang bei dieser Temperatur gehalten und dann auf ungefähr 20 bis 30°C abgekühlt. Die Suspension wird in einen 2-1-Tropftrichter gefüllt.

In einem 6-l-Planschliffkolben werden 3,5 l Wasser vorgelegt. Innerhalb von ungefähr 40 Minuten wird unter kräftigem Rühren und Eiskühlung das viskose Reaktionsgemisch aus dem Tropftrichter in das Wasser eingetragen. Die Temperatur wird dabei unter 40°C gehalten. Nach beendigter Zugabe wird noch 30 Minuten lang bei Raumtemperatur gerührt und das Produkt bei ungefähr 20°C abgesaugt. Es wird zweimal mit je 750 ml kaltem Wasser nachgewaschen (Sickerwäsche).

### 3.2 Freisetzung der Base (in Toluol)

In einem 6-l-Planschliffkolben werden 1,2 l Toluol, 0,8 1 Ethanol, 0,9 1 30%ige Natronlauge und 0,5 1 Wasser vorgelegt und durchgerührt. In dieses zweiphasige Gemisch wird unter Eiskühlung und kräftigem Rühren portionsweise das oben erhaltene Hydrogensulfat-Fumarsäure-Gemisch innerhalb von ungefähr 40 Minuten eingetragen. Nach der Zugabe wird die Suspension zum Sieden erhitzt (Innentemperatur ungefähr 75°C), wobei sich das ausgefallene Natriumfumarat weitgehend auflöst. Der Kolben wird mit Toluol (ungefähr 500 ml) gefüllt. Es wird kräftig durchgerührt. Dann werden in der Wärme die Phasen getrennt. Unlösliche Bestandteile an der Phasengrenzfläche werden mit der wäßrigen Phase abgetrennt. Die untere wäßrige Phase wird verworfen.

Aus der organischen Phase werden bei Normaldruck bis zu einer Innentemperatur von 116°C und einer Übergangstemperatur von 109°C Wasser, Ethanol und Toluol abdestilliert. Die im Rückstand verbleibende Toluol-Lösung von "Benzylmianserin"-Base wird direkt für die folgende Stufe eingesetzt.

Die Bestimmung des Gehalts der Lösung an "Benzylmianserin"-Base [Verbindung (VIII)] mit Perchlorsäure in wasserfreier Titration ergibt eine Ausbeute von 96% d. Th.

### 4. Herstellung von 1,2,3,4,10,14b-Hexahydro-dibenzo[c,f]pyrazino[1,2-a]azepin [Verbindung (XI)]

### 4.1 Debenzylierung zur Verbindung der Formel (X)

Die unter 3. erhaltene Lösung (ungefähr 900 ml) von "Benzylmianserin" in Toluol wird in einen 2-l-Dreihalskolben gefüllt. Unter Rühren werden bei ungefähr 60 bis 70°C Innentemperatur 213 ml Chlorameisensäurebutylester innerhalb von 20 Minuten zulaufen gelassen. Danach wird zum Sieden erhitzt und eine Stunde lang unter Rückfluß gehalten. Anschließend wird Toluol, überschüssiger Chlorameisensäurebutylester und entstandenes Benzylchlorid bis zu einer Sumpftemperatur von 155°C und einer Brückentemperatur bis 85°C im Wasserstrahlvakuum abdestilliert.

### 4.2 Carbamat-Spaltung der Zwischenverbindung (X)

Der unter 4.1 erhaltene Destillationsrückstand wird bei einer Innentemperatur von 80°C mit 300 ml n-Butanol versetzt und so lange gerührt bis eine homogene Lösung entstanden ist.

In einem 2-l-Dreihalskolben werden 290 g Kaliumhydroxid in 300 ml n-Butanol vorgelegt und zum Sieden erhitzt. Die obige Lösung von (X) in Butanol wird innerhalb von 30 Minuten unter Rückfluß zulaufen gelassen (Innentemperatur ungefähr 120°C). Anschließend wird das Gemisch 2 Stunden lang unter Rückfluß gehalten. Es wird auf 80°C abgekühlt, 500 ml warmes Wasser zulaufen gelassen und kräftig gerührt. Nach vollständiger Auflösung der ausgefallenen Salze werden die Phasen getrennt. Die Butanol-Lösung wird mit 300 ml warmen Wasser gewaschen. Zur Phasentrennung ist der Zusatz von Kochsalz nötig. Zur Entfernung gelöster Salze wird die Butanol-Lösung durch azeotrope Destillation bei Normaldruck entwässert. Abdestilliertes Butanol wird durch Zugabe der gleichen Menge an frischem Butanol ergänzt.

Die so erhaltene Lösung von 1,2,3,4,10,14b-Hexahydrodibenzo[c,f]pyrazino[1,2-a]azepin ("Normianserin") in Butanol (rotbraun) kann direkt für die Darstellung von Mianserin eingesetzt werden.

Die Bestimmung des Gehalts der Lösung an "Normianserin"-Base durch wasserfreie Titration mit Perchlorsäure ergibt eine Ausbeute von 88% d. Th.

### 4.3 Fällung von Normianserin-Hydrochlorid

Alternativ kann gemäß folgendem Verfahren das Hydrochlorid der Verbindung gewonnen werden:
Bei einer Anfangstemperatur von 20°C werden innerhalb von 10 Minuten 150 bis 160 ml konzentrierte Salzsäure zur Lösung von Normianserin-Base in n-Butanol (Vol.: ungefähr 900 ml) zulaufen gelassen. Der pH-Wert sollte ≤ 2 sein.

Eine Kristallisation des Normianserin-Hydrochlorids kann bereits nach Zugabe der Salzsäure auftreten. Bis zu einer Sumpftemperatur von 70°C wird n-Butanol und Wasser so lange abdestilliert bis die entstehende Suspension nicht mehr rührfähig ist. Dann wird wiederum mit 500 ml n-Butanol versetzt und so lange destilliert bis kein Wasser mehr übergeht. Die Suspension von Normianserin-Hydrochlorid in n-Butanol wird mit 600 ml Aceton versetzt und unter Rühren auf Raumtemperatur abgekühlt. Das Hydrochlorid wird abgesaugt, zweimal mit je 300 ml Aceton gewaschen (Sickerwäsche) und im Vakuum bei 60°C getrocknet.

Ausbeute: 368,8 g (85,7% d. Th.) an farblosen bis graustichigen Kristallen.

### 5. Herstellung von 1,2,3,4,10,14b-Hexahydro-2-methyl-dibenzo[c,f]pyrazino[1,2-a]azepin ["Mianserin", Verbindung (I)]

In einem 4-l-Vierhalskolben werden 230 ml Butanol vorgelegt und unter Rühren mit 230 ml 37%iger Formalin-Lösung versetzt. Dabei fällt die Temperatur von 20 auf 16°C. Zu diesem Gemisch läßt man zügig die unter 4. erhaltene "Normianserin"-Lösung in n-Butanol unter Rühren zulaufen (5 bis 10 Minuten). Dabei ist ein Temperaturanstieg bis auf maximal 30°C zu beobachten. Nach der Zugabe wird noch eine Stunde gerührt. Die erhaltene Lösung wird in einen 3-l-Edelstahlautoklaven gefüllt, mit wenig Butanol nachgespült und mit 15 ml konzentrierter Salzsäure versetzt.

Der Autoklav wird mit Stickstoff gespült und dann der mit Wasser angefeuchtete Katalysator (trocken 26 g, mit 70 ml Wasser angeteigt) eingetragen. Nach der Katalysatorzugabe werden auf den geschlossenen Autoklaven dreimal je 2 bar Stickstoff aufgedrückt. Anschließend wird jeweils wieder auf Normaldruck entspannt. Man heizt den Autoklaven auf ungefähr 60°C Innentemperatur auf, drückt dann 1 bis 1,5 bar Wasserstoff auf und hydriert bei diesem Druck. Der Katalysator wird über eine Drucknutsche abfiltriert. Autoklav und Nutsche werden zweimal mit je 80 ml Butanol nachgespült.

Von der so erhaltenen braunen, zweiphasigen Lösung destilliert man im Wasserstahlvakuum bis zu einer Badtemperatur von 60°C 1900 ml Wasser-Butanol-Gemisch ab. Der Rückstand wird bei ungefähr 60°C Innentemperatur unter Rühren mit 1350 ml Aceton versetzt. Bei 40°C wird zunächst 40 ml konzentrierte Salzsäure zugetropft (schwacher Temperaturanstieg auf ungefähr 43°C) und dann angeimpft.

Die wasserfreie Form des Mianserin-Hydrochlorids beginnt rasch zu kristallisieren. Dann wird die restliche Menge an Salzsäure (ungefähr 150 ml) innerhalb von ungefähr 15 Minuten zugetropft bis der pH-Wert ≤ 2 ist. Es wird zunächst mit kaltem Wasser abgekühlt, dann 2 Stunden lang im Eisbad gerührt, abgesaugt, dreimal mit je 150 ml Aceton gewaschen und gut trockengesaugt.

Zur Ausbeutebestimmung wird das rohe Mianserin-Hydrochlorid im Vakuum bei 80°C eine Stunde lang getrocknet. Für die weitere Reinigung kann es feucht eingesetzt werden.

Ausbeute: 429 g (95% d. Th.) cremefarbene Kristalle.

### Umkristallisation des Rohhydrochlorids

429 g Mianserin-Hydrochlorid (roh) werden in 2400 ml Ethanol (1:5,5) mit 8,5 g Aktivkohle und 10 g Supercel 5 Minuten unter Rückfluß erhitzt und anschließend klärfiltriert. Es wird mit 50 ml heißem Ethanol nachgewaschen.

Die Lösung ist braun. Das Mianserin-Hydrochlorid beginnt rasch zu kristallisieren. Es wird zunächst mit kaltem Wasser, dann im Eisbad bis ungefähr 5°C abgekühlt, abgesaugt und mit 220 ml kaltem Ethanol nachgewaschen. Das Produkt wird im Vakuum 4 Stunden bei 80°C getrocknet.

Ausbeute: 365 g (85% d. Th.)
Die Überprüfung der Reinheit mittels DC (Dichlormethan/ Methanol 90/10, Silicagel G = Methode der BP 88) zeigt keinerlei Verunreinigungen.

## Patentansprüche

1. Verfahren zur Herstellung von 1,2,3,4,10,14b-Hexahydro-2-methyl-dibenzo[c,f]pyrazino[1,2-a]azepin der Formel (I) 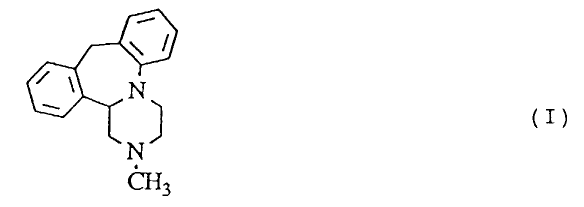 und seiner physiologisch annehmbaren Salze, ausgehend von Styroloxid, dadurch **gekennzeichnet,** daß
(a) Styroloxid der Formel (II) 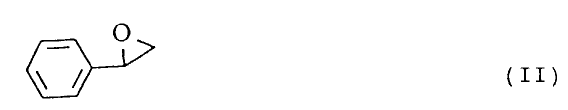 mit N-Benzylaminoethanol der Formel (III) 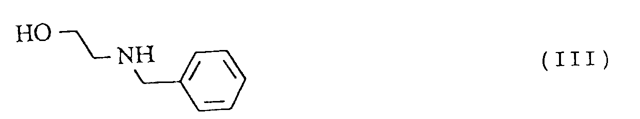 bei einer Reaktionstemperatur von 80 bis 160°C zu der Verbindung der Formel (IV) 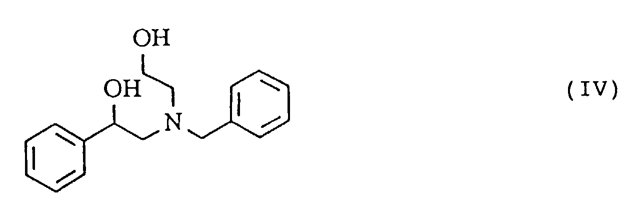 umgesetzt wird,
(b) die erhaltene Verbindung der Formel (IV) in einem inerten aprotischen Lösungsmittel mit einem Chlorierungsmittel in die Verbindung der Formel (V) 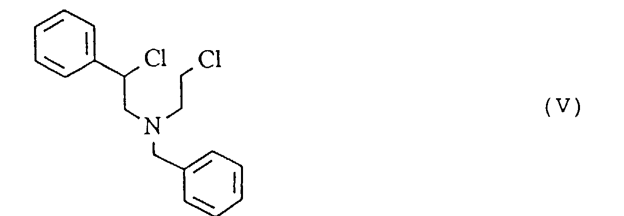 überführt wird und die resultierende Verbindung der Formel (V) ohne Isolierung der Verbindung der Formel (V) mit 2-Aminobenzylalkohol der Formel (VI) 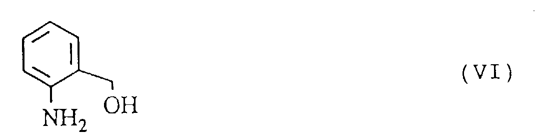 zu der Verbindung der Formel (VII) 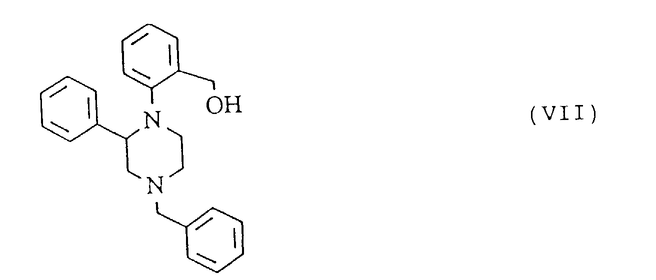 kondensiert wird, wobei das Molverhältnis des 2-Aminobenzylalkohols der Formel (VI) zu der Verbindung der Formel (V) 0,8:1 bis 1,2:1 beträgt, wobei die unter Cyclisierung erfolgende Kondensation in einem Zweiphasensystem erfolgt, wobei dem Reaktionsgemisch ein entsprechendes Äquivalent Base zur Bindung der bei der Reaktion entstehenden Säure zugesetzt wird und wobei die Isolierung der Verbindung der Formel (VII) durch Kristallisation eines geeigneten Salzes erfolgt,
(c) die erhaltene Verbindung der Formel (VII) mit Hilfe einer starken Säure, die in großem molaren Überschuß eingesetzt wird, zu der Verbindung der Formel (VIII) 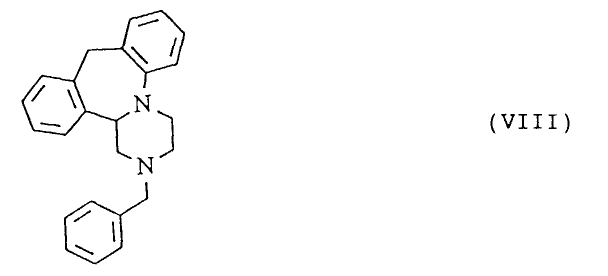 cyclisiert wird, wonach das zunächst gebildete Salz der Verbindung der Formel (VIII) zur Bildung der freien Base der Verbindung (VIII) mit einer geeigneten Base umgesetzt wird,
(d) in der erhaltenen Verbindung der Formel (VIII) die Benzylgruppe durch Umsetzung mit einem molaren Überschuß eines Chlorameisensäureesters der Formel (IX) 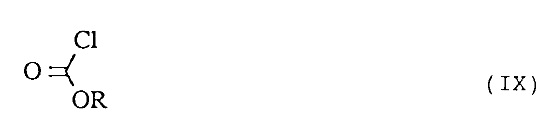 worin R für eine C₁-C₆-Alkylgruppe, eine Phenyl- oder Benzylgruppe steht, in einem aprotischen organischen Lösungsmittel entfernt und die intermediär gebildete Verbindung der Formel (X) 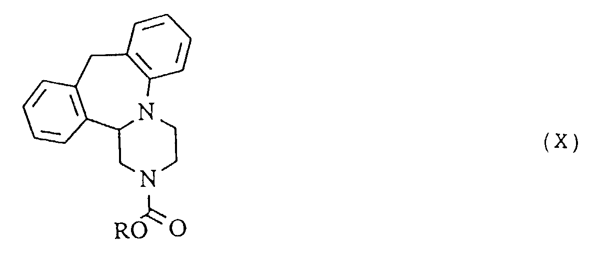 mit einer Base zu der Verbindung der Formel (XI) 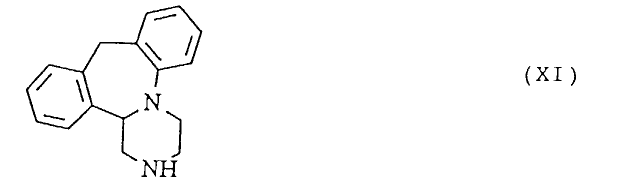 verseift wird
(e) die resultierende Verbindung der Formel (XI) unter reduzierenden Bedingungen mit Formaldehyd durch katalytische reduktive Aminierung mit Wasserstoff zu 1,2,3,4,10,14b-Hexahydro-2-methyl-dibenzo[c,f]pyrazino[1,2-a]azepin der Formel (I) methyliert wird, und
(f) gegebenenfalls die in Stufe (e) erhaltene Base in an sich bekannter Weise in ihr physiologisch annehmbares Salz umgewandelt wird.

2. Verfahren nach Anspruch 1, dadurch **gekennzeichnet**, daß man in Stufe (f) die Base in das Hydrochlorid umwandelt.

## Claims

1. A method of producing 1,2,3,4,10, 14b-hexahydro-2-methyl-dibenzo[c,f]pyrazino[1,2-a] azepine of formula (I) 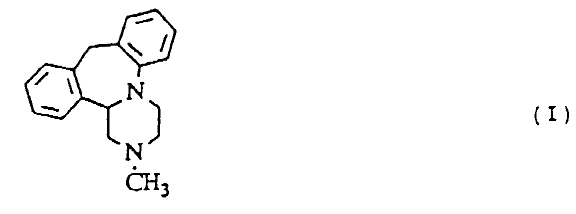 and physiologically acceptable salts thereof, starting from styrene oxide, characterised in that
(a) styrene oxide of formula (II) 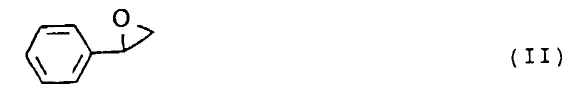 is reacted with N-benzylaminoethanol of formula (III) 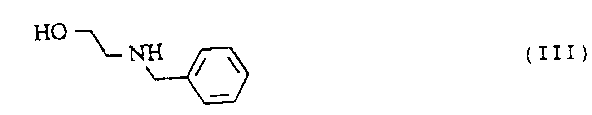 at a reaction temperature of 80 to 160°C to form the compound of formula (IV) 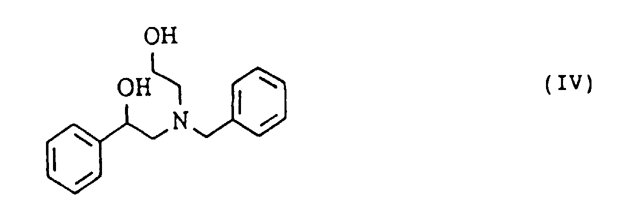
(b) the compound of formula (IV) which is obtained is converted, in an inert aprotic solvent with a chlorinating agent, into the compound of formula (V) 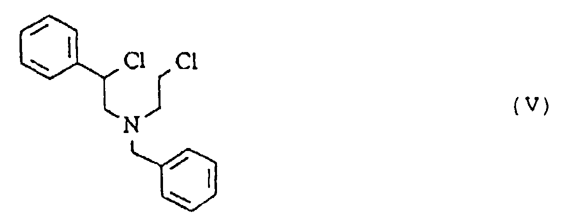 and the resulting compound of formula (V) is condensed with 2-aminobenzyl alcohol of formula (VI) 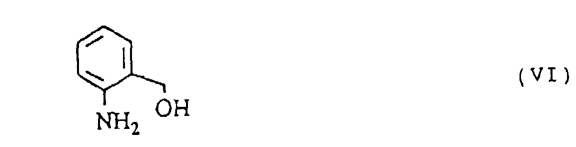 without isolating the compound of formula (V), to form the compound of formula (VII) 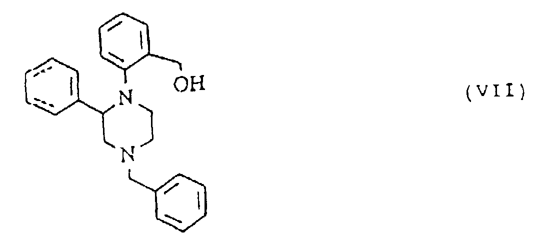 wherein the molar ratio of the 2-aminobenzyl alcohol of formula (VI) to the compound of formula (V) is 0.8:1 to 1.2:1, wherein the condensation, which occurs with cyclisation, is effected in a two-phase system, wherein a corresponding equivalent base is added to the reaction mixture for binding the acid formed during the reaction, and wherein isolation of the compound of formula (VII) is effected by the crystallisation of a suitable salt,
(c) the compound of formula (VII) which is obtained is cyclised, by means of a strong acid which is used in a large molar excess, to form the compound of formula (VIII) 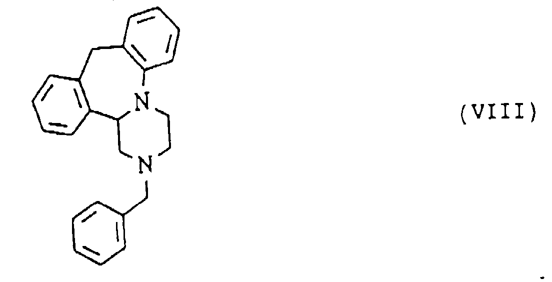 after which the salt of the compound of formula (VIII) which is first formed is reacted with a suitable base to form the free base of compound (VIII),
(d) in the compound of formula (VIII) which is obtained, the benzyl group is removed in an aprotic solvent by reaction with a molar excess of an ester of chloroformic acid of formula (IX) 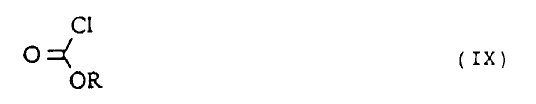 wherein R represents a C₁-C₆ alkyl group or a phenyl or benzyl group, and the compound of formula (X) 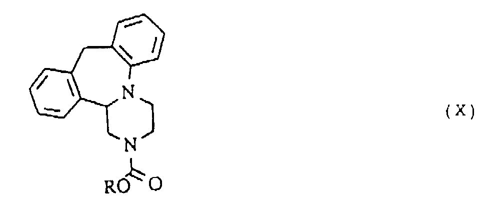 which is formed as an intermediate is saponified with a base to form the compound of formula (XI) 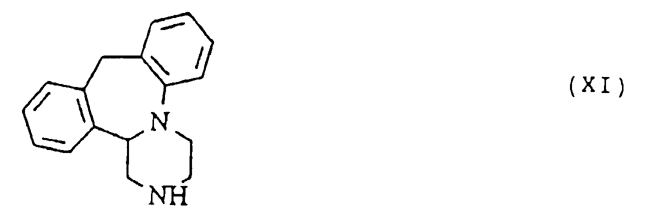
(e) the resulting compound of formula (XI) is methylated, under reducing conditions with formaldehyde, by catalytic reductive amination with hydrogen to form 1,2,3,4,10,14b-hexahydro-2-methyl-dibenzo[c,f]pyrazino[1,2-a]azepine of formula (I), and
(f) the base obtained in step (e) is optionally converted, in a manner which is known in the art, into a physiologically acceptable salt thereof.

2. A method according to claim 1, characterised in that in step (f) the base is converted into the hydrochloride.

## Revendications

1. Procédé de préparation de 1,2,3,4,10,14b-hexahydro-2-méthyl-dibenzo[c,f]pyrazino[1,2-a]azépine de formule (I) 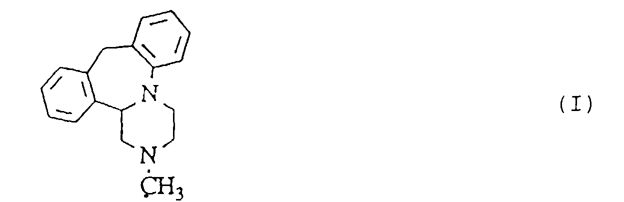 et de ses sels physiologiquement acceptables, à partir d'oxyde de styrène, caractérisé en ce que
(a) on fait réagir de l'oxyde de styrène de formule (II) 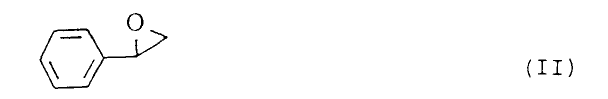 avec du N-benzylaminoéthanol de formule (III) 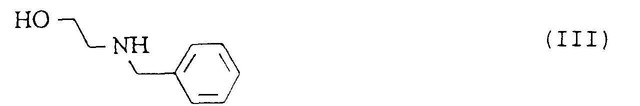 à une température de réaction de 80 à 160°C pour obtenir le composé de formule (IV) 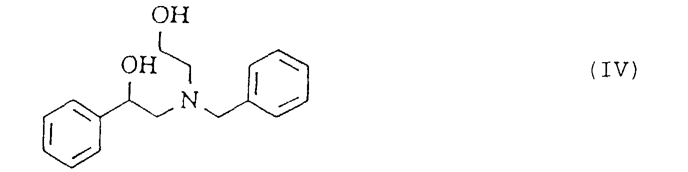
(b) on transforme le composé obtenu, de formule (IV), dans un solvant aprotique inerte avec un agent de chloration, en le composé de formule (V) 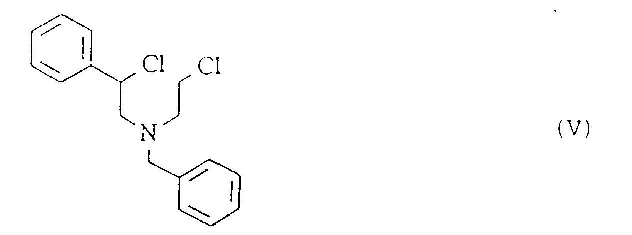 et on condense le composé résultant de formule (V), sans isolement du composé de formule (V), avec de l'alcool 2-aminobenzylique de formule (VI) 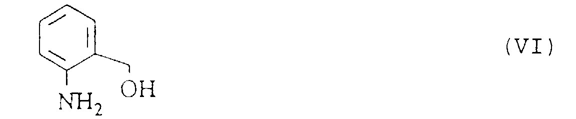 en le composé de formule (VII) 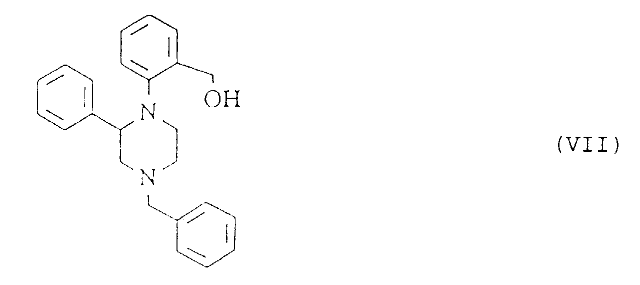 le rapport molaire de l'alcool 2-aminobenzylique de formule (VI) au composé de formule (V) étant de 0,8:1 à 1,2:1, la condensation effectuée avec cyclisation se déroulant dans un système à deux phases, on ajoute au mélange de réaction un équivalent correspondant d'une base pour fixer l'acide formé pendant la réaction et on effectue l'isolement du composé de formule (VII) par cristallisation d'un sel approprié,
(c) on cyclise le composé obtenu de formule (VII), à l'aide d'un acide fort qu'on utilise selon un excédent molaire élevé, en le composé de formule (VIII) 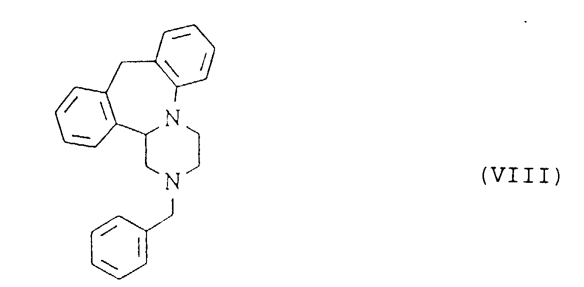 après quoi on fait réagir le sel d'abord formé du composé de formule (VIII) avec une base appropriée pour la formation de la base libre du composé (VIII),
(d) dans le composé obtenu de formule (VIII), on élimine le groupe benzyle par réaction avec un excès molaire d'un ester d'acide chlorofomique de formule (IX) 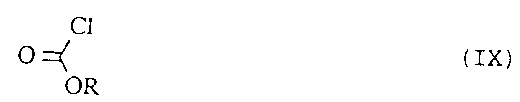 où R représente un groupe alkyle en C₁-C₆, le groupe phényle ou le groupe benzyle, dans un solvant organique aprotique, et on saponifie le composé intermédiaire formé, de formule (X) 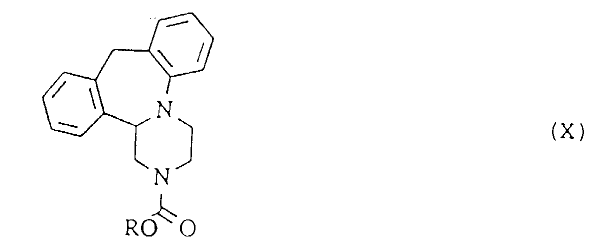 avec une base pour obtenir le composé de formule (XI) 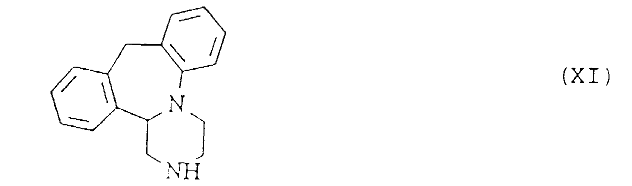
(e) on effectue la méthylation du composé résultant de formule (XI), dans des conditions de réduction, avec du formaldéhyde, par amination catalytique réductrice avec de l'hydrogène pour obtenir la 1,2,3,4,10,14b-hexahydro-2-méthyl-dibenzo[c,f]pyrazino[1,2-a]azépine de formule (I) et
(f) éventuellement, on transforme la base obtenue lors de l'étape (e), d'une manière connue en soi, en son sel physiologiquement acceptable.

2. Procédé selon la revendication 1, caractérisé en ce qu'on transforme la base en chlorhydrate lors de l'étape (f).
